# EUROPÄISCHE PATENTSCHRIFT

(11) **EP 0 363 942 B1**
(45) Veröffentlichungstag und Bekanntmachung des Hinweises auf die Patenterteilung: **16.02.1994**
(21) Anmeldenummer: 89118917.7
(22) Anmeldetag: 11.10.1989
(51) Int. Cl.: G01N 33/543, G01N 33/78

(54) **Verfahren zur Bestimmung einer spezifisch bindefähigen Substanz**
Method for the determination of a specific binding substance
Procédé pour la détermination d'une substance liante spécifique

(30) Priorität: 12.10.1988 DE 3834766
(43) Veröffentlichungstag der Anmeldung: 18.04.1990
(73) Patentinhaber: BOEHRINGER MANNHEIM GMBH, 68298 Mannheim (DE)
(72) Erfinder: Schenk, Roland, Dr.rer.nat., D-8120 Weilheim (DE)
(74) Vertreter: Huber, Bernhard, Dipl.-Chem.

(56) Entgegenhaltungen:
- GB-A- 2 084 317
- US-E- 32 696

## Beschreibung

Die Erfindung betrifft ein Verfahren zur Bestimmung einer spezifisch bindefähigen Substanz durch Inkubation der Probelösung mit mindestens drei Rezeptoren R₁, R₂ und R₃, von denen R₁ mit R₂ und R₃ sowie mit der zu bestimmenden Substanz spezifisch bindefähig ist, R₂ die Bindung an die feste Phase vermittelt und R₃ eine Markierung trägt, Abtrennung gebundener von ungebundener Markierung und Messung der Markierung in einer der beiden Phasen sowie ein geeignetes Reagenz.

In Körperflüssigkeiten und Geweben kommen sehr viele Substanzen vor, die immunologisch aktiv sind, d.h. die mit einem spezifischen Bindungspartner bindefähig sind und als Parameter für bestimmte Erkrankungen oder den Gesundheitszustand des menschlichen Körpers dienen. Hierzu zählen unter anderem Haptene wie z. B. Hormone, Proteine wie Tumormarker, Proteinhormone und virale Proteine sowie Antikörper. Auch ist zur Überwachung einer medikamentösen Behandlung häufig die Bestimmung von Arzneistoffen im Blut erforderlich. Beispiele hierfür sind Antiepileptika, Antibiotika, Digitalis und Opiate. Da diese Substanzen oft nur in sehr geringen Mengen vorkommen, verwendet man zu ihrem Nachweis Verfahren nach dem Prinzip des Immunoassays. Dazu gibt es viele Varianten. Die verschiedenen immunologischen Bestimmungsverfahren lassen sich in homogene und heterogene Verfahren einteilen. Bei den heterogenen Verfahren ist immer eine Festphasenreaktion beteiligt, um den gebundenen Anteil der markierten Komponente von dem ungebundenen abzutrennen. Bei der homogenen Verfahrensvariante erfolgt keine Trennung von gebundener Markierung und ungebundener Markierung, so daß eine Differenzierung von gebundener und ungebundener Markierung durch andere Methoden erfolgen muß.

Zur Durchführung von heterogenen Immunoassays gibt es im wesentlichen zwei Varianten, wobei einmal ein Antikörper gegen die zu bestimmende Substanz immobilisiert wird und das anderemal die zu bestimmende Substanz selbst immobilisiert wird. In der ersten Variante wird eine Probelösung, die die zu bestimmende Substanz enthält, und ein Konjugat aus zu bestimmender Substanz und einer Markierung mit dem immobilisierten Antikörper inkubiert. Dabei konkurrieren zu bestimmende Substanz und markierte Substanz um die Bindung an den Antikörper. Je mehr zu bestimmende Substanz in der Lösung vorhanden ist, desto weniger markierte Substanz kann gebunden werden. Die Menge an gebundener markierter Substanz ist daher ein indirektes Maß für die Menge an zu bestimmender Substanz. Nach der Trennung von fester und flüssiger Phase kann dann die Markierung in einer der beiden Phasen bestimmt werden.

In der anderen Variante wird die Probelösung, die die zu bestimmende Substanz enthält, mit einem für sie spezifischen markierten Antikörper sowie der immobilisierten Probensubstanz inkubiert. Hierbei konkurrieren immobilisierte Probensubstanz und in der Lösung vorhandene zu bestimmende Substanz um die Bindung an den markierten Antikörper. Je mehr zu bestimmende Substanz in der Lösung vorhanden ist, desto weniger markierter Antikörper wird durch Bindung an die immobilisierte Probensubstanz an der Festphase gebunden. Die Menge an gebundenem markiertem Antikörper ist somit ebenfalls ein indirektes Maß für die Menge an zu bestimmender Substanz in der Probelösung. Auch hier wird wiederum nach Trennung der festen von der flüssigen Phase die Menge an gebundener Markierung bestimmt.

Der Nachteil dieser Verfahren ist es, daß immer modifizierte Antikörper verwendet werden müssen. Im ersten Fall wird der Antikörper an eine Festphase gebunden, im zweiten Fall wird der Antikörper an eine Markierungssubstanz gebunden. Diese Modifizierung ist aufwendig und kann zu unerwünschten Änderungen der Eigenschaften der Antikörper, wie z.B. Verringerung der Spezifität und Affinität, führen. Dies ist der Grund dafür, daß häufig nicht polyklonale Antikörper verwendet werden können, sondern mit großem Aufwand auf geringe Kreuzreaktivität und hohe Affinität selektierte monoklonale Antikörper eingesetzt werden müssen, die zudem meist noch auf aufwendige Art und Weise gereinigt werden müssen. Außerdem müssen für jede einzelne Bestimmung alle Reagenzien spezifisch ausgewählt werden, was ebenfalls ungünstig ist. Zwar war es bereits aus DE-OS 31 38 489 bekannt, die Immobilisierung gebildeter Markierungskomplexe ohne Beteiligung von Antikörpern durchzuführen, jedoch ist auch bei diesem Verfahren die Verwendung eines markierten und damit modifizierten Antikörpers erforderlich.

Aufgabe der vorliegenden Erfindung war es deshalb, ein Verfahren zur Bestimmung von immunologisch aktiven Substanzen bereitzustellen, das vielseitig verwendbar ist, bei dem Antikörper ohne Modifizierung verwendet werden können und bei dem nicht unbedingt monoklonale Antikörper eingesetzt werden müssen.

Diese Aufgabe wird gelöst durch ein Verfahren zur Bestimmung einer spezifisch bindefähigen Substanz durch Inkubation mit drei Rezeptoren R₁, R₂ und R₃, von denen R₁ mit R₂ und R₃, sowie mit der zu bestimmenden Substanz spezifisch bindefähig ist, R₂ die Bindung an die feste Phase vermittelt und R₃ eine Markierung trägt, Abtrennung gebundener von ungebundener Markierung und Messung der Markierung in einer der beiden Phasen, welches dadurch gekennzeichnet ist, daß als Rezeptor R₁ ein Rezeptor verwendet wird, der mindestens zwei Bindungsstellen, die spezifisch mit einem Epitop der zu bestimmenden Substanz binden, aufweist, R₂ ein Konjugat aus einem Partner P1 eines spezifisch bindenden Paares und einer Substanz S, die der zu bestimmenden Substanz entspricht oder ein Derivat davon ist und mindestens ein Epitop der zu bestimmenden Substanz aufweist, wobei der Partner P1 entweder an eine feste Phase gebunden oder immobilisierbar ist und als R₃ ein Komplex verwendet wird, der mindestens die Substanz S und eine Markierung enthält, verwendet wird.

Das erfindungsgemäße Verfahren ist geeignet zur Bestimmung praktisch aller in Körperflüssigkeiten oder Gewebeextrakten nachzuweisenden zu einer spezifischen Bindung befähigten Substanzen, wobei niedrigkonzentrierte Substanzen ebenso gut nachweisbar sind wie hochkonzentrierte. Die Empfindlichkeit und Genauigkeit des Verfahrens ist gegenüber den bisher bekannten Verfahren verbessert. Die Erfindung bietet die Möglichkeit, mit einfachen Reagenzien schnell und zuverlässig Bestimmungen durchzuführen.

Das Verfahren ist insbesondere geeignet zur Bestimmung von Haptenen, d.h. Substanzen, die für einen spezifisch bindefähigen Partner nur eine Bindungsstelle besitzen. Als Beispiele sind hier Hormone und Arzneistoffe wie Antiepileptika, Antiobiotika, Digitalis und Opiate zu nennen.

Es wurde überraschenderweise gefunden, daß bei Durchführung des erfindungsgemäßen Verfahrens im Gegensatz zu dem bekannten Verfahren an die Qualität der Antikörper keine hohen Ansprüche zu stellen sind, so daß hier ohne weiteres polyklonale Antikörper verwendet werden können. Darüberhinaus wurde festgestellt, daß die erhaltenen Leerwerte nur sehr gering sind und damit die Empfindlichkeit gegenüber bekannten Verfahren erhöht ist.

Unter Epitop wird in der Beschreibung der vorliegenden Erfindung eine Bindungsstelle verstanden, die eine spezifische Bindung mit einer anderen Substanz eingehen kann. Beispiele für Epitope sind antigene Determinanten auf Antigenen und Haptenen oder auch spezifische Bindungsstellen auf Proteinen.

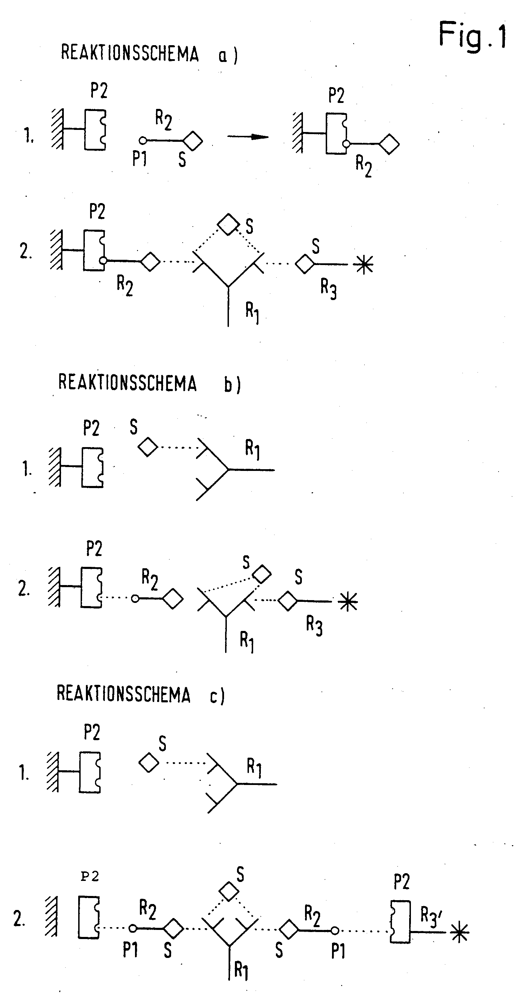

Zur Durchführung des erfindungsgemäßen Verfahrens wird die Probelösung mit drei Rezeptoren R₁, R₂ und R₃ inkubiert. Rezeptor R₁ ist dabei mit den Rezeptoren R₂ und R₃ sowie der zu bestimmenden Substanz spezifisch bindefähig und trägt eine Markierung. Rezeptor R₂ vermittelt die Bindung an die feste Phase. Verschiedene Reaktionsprinzipien, die mit dem erfindungsgemäßen Verfahren durchgeführt werden können, sind in der Fig. 1 dargestellt. Eine bevorzugte Verfahrensvariante ist die als 1a) bezeichnete Durchführungsform. Dabei wird in ein Reaktionsgefäß, an dessen Wand eine Vielzahl von zu P1 komplementären Partnern P2 eines spezifisch bindenden Paares gebunden sind, Rezeptor R₂, der ein Konjugat aus der Substanz S und dem Partner P1 des spezifisch bindenden Paares ist, zugegeben. Das Konjugat bindet über Partner P1 an Partner P2. Anschließend wird in das Reaktionsgefäß Rezeptor R₁, der mindestens zwei Bindungsstellen für ein Epitop der zu bestimmenden Substanz aufweist und Rezeptor R₃, der ein Konjugat aus der Substanz S und einer Markierung ist, zugegeben. In der Lösung konkurrieren dann der Teil S von R₂, der Teil S von R₃ und die zu bestimmende Substanz um Bindung an Rezeptor R₁. Ist R₁ beispielsweise ein bivalenter Antikörper, so bilden sich die folgenden Komplexe:
- R₁,: an dessen beiden Paratopen jeweils R₂ über S gebunden ist,
- R₁,: an dessen beiden Paratopen die zu bestimmende Substanz gebunden ist,
- R₁,: an dessen beiden Paratopen R₃ über S gebunden ist, sowie gemischte Komplexe und zwar:
- R₁,: an dessen einem Paratop die zu bestimmende Substanz und an dessen anderem Paratop R₃ oder R₂ über S gebunden ist und
- R₁,: an dessen einem Paratop R₃ über S und an dessen anderem Paratop R₂ über S gebunden ist.

Nur die Komplexe, die an R₂ gebunden sind, werden immobilisiert und nur die Komplexe, bei denen ein Rezeptor R₃ gebunden ist, können über die Bestimmung der gebundenen Markierung ausgewertet werden. Je mehr von der zu bestimmenden Substanz in der Lösung enthalten ist, desto weniger R₂ und R₃ werden an R₁ gebunden, desto weniger Komplexe werden also immobilisiert bzw. tragen eine Markierung. Damit ist der Anteil an gebundener Markierung ein indirektes Maß für die zu bestimmende Substanz und kann über eine Eichkurve ausgewertet werden.

Die Durchführungsvariante b) dient zum Nachweis von Substanzen, die in sehr geringer Konzentration in der Probelösung vorhanden sind. Hier wird im ersten Schritt die Matrix, an der Partner P2 des spezifisch bindenden Paares gebunden sind, mit der zu bestimmenden Substanz sowie Rezeptor R1 inkubiert. Dabei kann in der Probelösung vorhandene zu bestimmende Substanz mit Rezeptor R1 binden. Eine Bindung an die Festphase kann nicht erfolgen. In einem zweiten Schritt werden dann Rezeptoren R₂ und R₃ zugegeben. Die zugegebenen Rezeptoren konkurrieren dann um die Bindung an Rezeptor R₁, an dem teilweise schon die zu bestimmende Substanz gebunden ist. Je mehr von der zu bestimmenden Substanz bereits gebunden ist, desto weniger Rezeptoren R₂ und R₁ können gebunden werden. Da Rezeptor R₂ weiterhin Partner P1 enthält, der mit Partner P2 der Matrix bindefähig ist, kommt es zur Immobilisierung der sich bildenden Komplexe. Die Auswertung erfolgt wiederum nach Abtrennung gebundener von flüssiger Phase durch Auswertung der Markierung, die über eine Eichkurve erfolgt.

Variante c) zeigt eine Durchführungsform, bei der die die Festphase bildende Matrix und das Markierungskonjugat für alle Bestimmungen gleich bleiben können. Hier wird wiederum in einem ersten Schritt die Matrix, die Partner P2 des spezifisch bindenden Paares trägt, mit der zu bestimmenden Substanz und Rezeptor R₁ inkubiert, wobei die zu bestimmende Substanz an Rezeptor R₁ binden kann. Im zweiten Schritt wird dann gleichzeitig das Konjugat R₂ aus der zu bestimmenden Substanz und Partner P1 und ein Konjugat R₃′ aus Partner P2 und einer Markierung zugegeben. Es konkurrieren dann das Konjugat R₂ aus zu bestimmender Substanz und Partner P1 sowie die zu bestimmende Substanz aus der Probelösung um Bindung an Rezeptor R₁. Weiterhin konkurrieren der an der Matrix gebundene Partner P2 sowie das Konjugat R₃′ aus Partner P2 und Markierung um die Bindung an einen der Partner P1. Auch hier erfolgt wieder eine Auswertung der gebundenen markierten Komplexe über eine Eichkurve.

Bei dieser Variante werden die Konzentrationen von R₁, also insbesondere des Antikörpers und von R₂ auf die Probenkonzentration im Test abgestimmt. Das Konjugat aus Partner P2 und einer Markierung sollte dabei zweckmäßig in einer Menge eingesetzt werden, die nicht mehr als etwa die Hälfte des Konjugats R₂ aus der zu bestimmenden Substanz und Partner P1 bindet, damit von letzterem Konjugat genügend für die Bindung am matrixgebundenen Partner P2, der im Überschuß vorliegen sollte, zur Verfügung steht. Das P1 enthaltende Konjugat R₂ wird zweckmäßig in einer Menge verwendet, die in der Größenordnung der Menge der zu bestimmenden Substanz liegt, also etwa 10⁻⁵ mol/l (z.B. bei Theophyllin) bis 10⁻¹⁰ mol/l (z.B. bei Digoxin). Bei Variante c) darf R₂ nur eine Bindungsstelle für P2 (an der Matrix oder im Konjugat) haben, um eine störende Vernetzung zu vermeiden. Dies gilt nicht für die Varianten a) und b).

Diese Ausführungsform hat zum einen den großen Vorteil, daß nur ein einziges spezifisches Konjugat - das Konjugat aus S und P1 - zur Verfügung gestellt werden muß und zum anderen läuft die immunologische Reaktion homogen ab, was Empfindlichkeit und Reproduzierbarkeit deutlich erhöht.

Für das erfindungsgemäß definierte Verfahrensprinzip gibt es also viele Durchführungsvarianten. In jedem Fall sind mindestens drei Rezeptoren erforderlich.

Die zu bestimmende Substanz kann jede zu einer spezifischen Bindung fähige Substanz sein und insbesondere wie oben definiert ein Hapten sein.

Als erster Rezeptor R₁ wird ein Rezeptor verwendet, der mindestens zwei Bindungsstellen, die spezifisch mit einem Epitop der zu bestimmenden Substanz binden, aufweist. Dieser Rezeptor wird nach der jeweils zu bestimmenden Substanz ausgewählt. Bevorzugt wird hier ein monoklonaler oder polyklonaler Antikörper oder deren Fab₂-Fragment verwendet.

Rezeptor R₂ ist ein Konjugat aus einem Partner eines spezifisch bindenden Paares P1 und einer Substanz S, die der zu bestimmenden Substanz entspricht oder ein Derivat davon ist und mindestens ein Epitop der zu bestimmenden Substanz aufweist. Spezifisch miteinander bindende Paare sind an sich bekannt. Geeignete Bindungspaare (P1-P2) sind insbesondere Biotin-Streptavidin bzw. Avidin; Hapten-Antikörper; Antigen-Antikörper; Konkanavalin-Antikörper; Zucker-Lectin; Hapten-Bindeprotein, z. B. Thyroxin bindendes Globulin und Thyroxin oder Oligopeptid-Antikörper.

Besonders bevorzugt wird als bindendes Paar Biotin und Streptavidin bzw. Avidin eingesetzt, so daß Rezeptor R₂ besonders bevorzugt Biotin als Partner P1 enthält.

Der Anteil S des Rezeptors R₂ kann bevorzugt der unveränderten zu bestimmenden Substanz entsprechen. Es kann auch ein Derivat der zu bestimmenden Substanz bzw. ein Teil der zu bestimmenden Substanz wie beispielsweise ein Proteinepitop verwendet werden. Wesentlich ist nur, daß der Anteil S mit Rezeptor R₁ bindefähig ist, wobei es nicht unbedingt erforderlich ist, daß S und die zu bestimmende Substanz mit der gleichen Bindungsstärke an R₁ binden.

Die Herstellung der Konjugate erfolgt in an sich bekannter Weise (z. B. analog Eur. J. Biochem. 131 (1980) 333-338).

Rezeptor R₂ vermittelt die Bindung an die feste Phase. R₂ kann in einer Variante direkt oder über einen Spacer an eine Festphase über P1 gebunden sein. In einer anderen Variante erfolgt die Bindung an die Festphase während der immunologischen Reaktion durch Bindung von P1 an an der Festphase immobilisierte Partner P2 des spezifisch bindenden Paares.

In einer weiteren bevorzugten Ausführungsform des Verfahrens wird Rezeptor R₂ verwendet, der ein Konjugat aus S und P1 ist. Als Festphase wird eine Matrix verwendet, an der ebenfalls eine Vielzahl von Partnern P1 des spezifisch bindenden Paares immobilisiert sind. Nach der Inkubation der für die immunologische Reaktion erforderlichen Komponenten wird dann Partner P2, der mindestens 2 Bindungsstellen für P1 aufweist, zugegeben. Auf diese Weise erfolgt dann die Immobilisierung der Rezeptoren R2.

Als feste Phase besonders geeignet sind Reagenzgläschen oder Mikrotiterplatten aus Polystyrol und ähnlichen Kunststoffen, die adsorptiv an der Innenoberfläche mit P1 bzw. P2 beschichtet sind. Weiterhin geeignet sind auch teilchenförmige Substanzen, z.B. Molekularsiebmaterialien, Glasperlen, Kunststoffschläuche und dergleichen. Ebenfalls geeignet sind als feste Phase auch poröse, schichtförmige Träger wie Papier. Bevorzugt ist auch eine Ausführungsform, in der die feste Phase aus magnetischen Partikeln besteht, wie sie beispielsweise in EP-A 0 240 770, US 4,141,687 und US 4,197,337 beschrieben sind. Die magnetischen Partikel bestehen vorzugsweise aus Chromdioxid oder Eisenoxid. Die Bindung des Partners P2 an die Partikel kann beispielsweise, wie in EP-A 0 240 770 beschrieben, erfolgen.

Rezeptor R₃ ist ein Komplex, der mindestens die Substanz S und eine Markierung enthält. Als Markierung wird dabei ein Enzym, eine fluoreszierende, chemilumineszierende oder radioaktive Substanz verwendet. Verfahren zur Markierung sind dem Fachmann bekannt, z.B. aus Clin. Chim. Acta 81 (1977) 1-40, und bedürfen hier keiner weiteren Erläuterung. Die Markierung kann in an sich bekannter Weise bestimmt werden.

Rezeptor R₃ kann auch erst während der immunologischen Reaktion gebildet werden aus einem Konjugat, das die Substanz S und einen Partner P1 eines spezifisch bindenden Paares enthält und einem Konjugat des Partners P2 des spezifisch bindenden Paares und einer Markierung.

Das Verfahren kann in einer oder mehr Stufen durchgeführt werden. Die Auswertung erfolgt in an sich bekannter Weise. Da jeder der Rezeptoren und auch die zu bestimmende Substanz jeweils nur spezifisch mit dem für ihn bestimmten Reaktionspartner reagieren kann, ist es möglich, alle Rezeptoren und die Probe zusammen zu inkubieren und das Verfahren einstufig durchzuführen. Dies ist besonders vorteilhaft bei der Durchführung des Verfahrens in einem Analysenautomaten.

Für den Nachweis von sehr niedrigkonzentrierten oder sehr hochkonzentrierten Substanzen sind mehrstufige Verfahrensvarianten bevorzugt.

Die Durchführung aller Verfahrensvarianten erfolgt vorzugsweise in einer gepufferten Lösung. Puffersysteme für diese Verfahren sind an sich bekannt. Besonders geeignet sind hierfür GOOD-Puffer und Phosphatpuffer.

Erfindungsgemäß wird ein Verfahren zur Verfügung gestellt, das einfach und schnell durchzuführen ist und auch bei Verwendung polyklonaler Antikörper sehr empfindlich ist.

Ein weiterer Gegenstand der vorliegenden Erfindung ist ein Reagenz zur Bestimmung von spezifisch bindefähigen Substanzen, welches dadurch gekennzeichnet ist, daß es Rezeptor R₁, der mindestens zwei für die zu bestimmende Substanz spezifische Bindungsstellen aufweist, Rezeptor R₂, der ein Konjugat aus einem Partner P1 eines spezifische bindenden Paares und einer Substanz S, die der zu bestimmenden Substanz entspricht, oder ein Derivat davon ist und mindestens ein Epitop der zu bestimmenden Substanz aufweist, ist und Rezeptor R₃, der ein Komplex ist, der mindestens eine Markierung und die Substanz S enthält und physikalisch getrennt davon eine Festphase enthält.

In einer weiteren Ausführungsform enthält das erfindungsgemäße Reagenz eine Festphase, an die eine Vielzahl Partner P2 eines spezifisch bindenden Paares gebunden sind, sowie physikalisch getrennt davon die Rezeptoren R₁, R₂ und R₃ oder R₃′.

In einer weiteren Ausführungsform enthält das erfindungsgemäße Reagenz eine Festphase, an der Partner P1 gebunden sind und physikalisch getrennt davon Partner P2 des spezifisch bindenden Paares sowie die Rezeptoren R₁, R₂ und R₃ oder R₃′.

In einer weiteren Ausführungsform enthält das Reagenz einen mehrschichtigen Reagenzträger, der in einer ersten Schicht die Festphase mit Partner P₂ und Rezeptor R₃ und physikalisch getrennt von dieser ersten Schicht die Rezeptoren R₁ und R₃ enthält.

In einer weiteren bevorzugten Ausführungsform wird eine Reagenzienkombination verwendet, die in einem ersten Reagenz eine Suspension der mit Partner P2 beschichteten magnetischen Partikel und eine Lösung des Rezeptors R₃′ (Reaktionsschema 1c) enthält. Ein weiteres Reagenz enthält die Rezeptoren R₁ und R₂. Vorzugsweise wird als spezifisch bindendes Paar für P₁/P₂ Biotin/Avidin bzw. Streptavidin verwendet.

Dieses Reagenz ist zur Bestimmung einer Vielzahl von Parametern in Körperflüssigkeiten und Gewebeextrakten geeignet.

In einer bevorzugten Ausführungsform enthält das Reagenz zusätzlich Puffersubstanzen. Besonders bevorzugt enthält es Phosphatpuffer oder GOOD-Puffer.

Die Erfindung wird durch die Figur und die Beispiele erläutert.
- Fig. 1: zeigt drei Reaktionsschemata für bevorzugte Ausführungsformen des erfindungsgemäßen Verfahrens.
- Fig. 2: zeigt eine Eichkurve für eine T₄-Bestimmung, unter Verwendung eines T₄-POD-Konjugats (Reaktionsschema 1a). Kurve 1: 10 nmol/l T₄-Biotin, Kurve 2: 1 nmol/l T₄-Biotin. (Endkonzentrationen im Test)
- Fig. 3: zeigt eine Eichkurve für eine T₄-Bestimmung, unter Verwendung eines T₄-POD-Konjugats (Reaktionsschema 1b).
- Fig. 4: zeigt eine Eichkurve für eine T₄-Bestimmung, unter Verwendung eines T₄-Biotinkonjugats und eines Streptavidin-POD-Konjugats (Reaktionsschema 1c).

In Figur 1 sind Schemata für die Reaktionsprinzipien verschiedener bevorzugter Ausführungsformen des erfindungsgemäßen Verfahrens gezeigt. In allen gezeigten Varianten ist Rezeptor R₁ ein Antikörper.

Figur 1a zeigt eine Variante, bei der zuerst eine Festphase, an die Partner P2 eines spezifisch bindenden Paares gebunden sind, mit Rezeptor R₂, der Partner P1 des spezifisch bindenden Paares aufweist, inkubiert wird, wobei eine Festphase, an der Rezeptoren R₂ über P1 gebunden sind, entsteht. In einem zweiten Schritt wird dann diese Festphase mit Rezeptor R₁, R₃ und der Probelösung inkubiert.

Variante 1b zeigt eine Ausführungsform, bei der in einem ersten Schritt eine Festphase, an der Partner P2 gebunden sind, mit Rezeptor R₁ und der Probelösung inkubiert werden. In einem zweiten Schritt werden Rezeptoren R₂ und R₁ zugegeben.

Variante 1c zeigt eine Ausführungsform, bei der eine Festphase, an der Partner P2 gebunden sind und Konjugate von Markierung und Partner P2 verwendet werden, die jeweils unabhängig von der zu bestimmenden Substanz sind. Mit Festphase und Markierungskonjugat werden die Rezeptoren R₁, R₂ und R₃, sowie die Probe inkubiert.

### Beispiel 1

### Bestimmung von T₄ nach Reaktionsschema 1a)

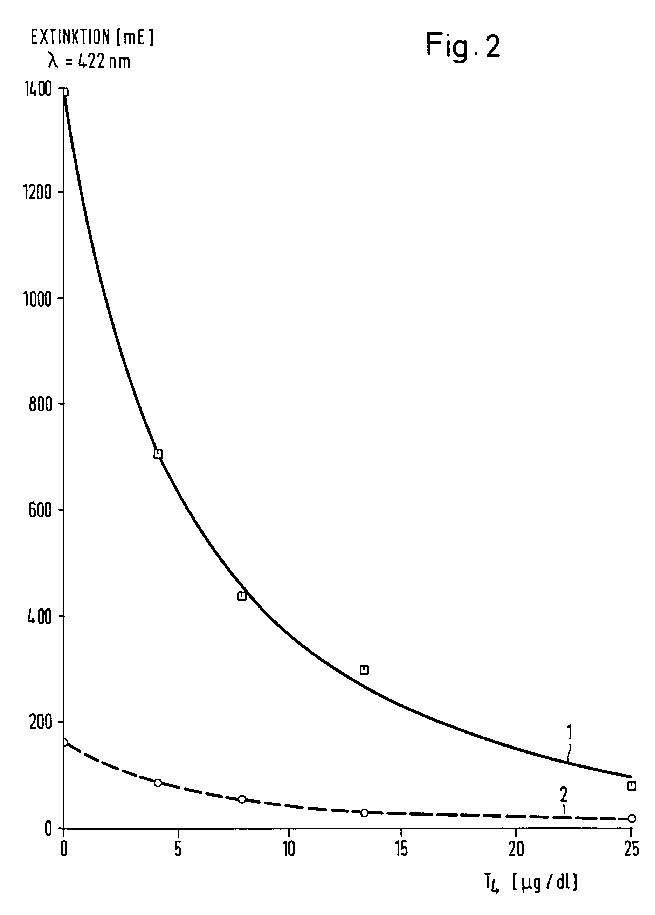

Puffer A:
120 mmol/l Barbiturat
18,2 mmol/l Phosphatpuffer, pH 8,6
1,27 mmol/l 8-Anilino-1-Naphthalinsulfonsäure
0,2 Gew.-% Rinderserumalbumin
980 µl Puffer A und 20 µl einer Lösung eines Konjugats aus T₄ und Biotin (Herstellung nach Beispiel 4 (Endkonzentration: 1 bzw. 10 mmol/ml)) werden in ein streptavidinbeschichtetes Polystyrolgefäß (Herstellung nach EP-A-0269092) gegeben und 30 Minuten bei 25°C inkubiert. Anschließend wird gewaschen und 50 µl Probe (Humanserum aufgestockt mit T₄), 980 µl Puffer A, 20 µl einer Lösung von 0,1 mg/ml polyklonalem Antikörper gegen T₄ und 2,5 U/ml T₄-Peroxidase-Konjugat (50 mU/Test) zugegeben, 30 Minuten bei 25°C inkubiert, gewaschen und 1 ml einer Lösung von 9,1 mmol/l ABTS^{R} (2,2′-Azino-di-(3ethylbenzthiazolin- sulfonsäure(6))-diammoniumsalz zugegeben. Nach einer weiteren Inkubation über 30 Minuten bei 25°C wird als Maß für den T₄-Gehalt die optische Dichte bei 422 nm bestimmt. Die Ergebnisse sind Figur 2 zu entnehmen.

### Beispiel 2

### Bestimmung von T₄ nach Reaktionsschema 1b

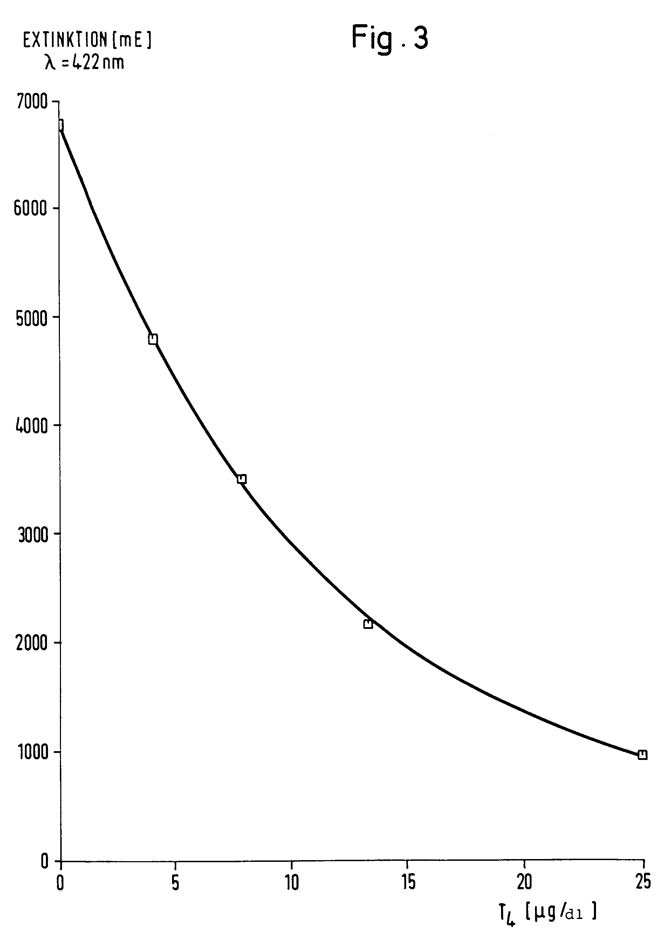

50 µl Probe (mit T₄ aufgestocktes Humanserum) werden mit 480 µl Puffer A und 20 µl einer Lösung eines polyklonalen Antikörpers gegen T₄ (0,1 mg/ml) bei 25°C 30 Minuten in einem streptavidinbeschichteten Polystyrolgefäß inkubiert. Anschließend werden 480 µl Puffer A und 20 µl einer Lösung von T₄-Biotin-Konjugat (5 x 10⁻⁸ mol/l, entspricht 1 nmol/l Endkonzentration im Test) und T₄-POD-Konjugat (2,5 U/ml, 50 mU/ Test) zugegeben und 30 Minuten bei 25°C inkubiert. Es wird gewaschen und anschließend 1 ml ABTS^{R}-Lösung (9,1 mmol/l) zugegeben, 30 Minuten bei 25°C inkubiert und anschließend bei 422 nm die optische Dichte als Maß für den T₄-Gehalt bestimmt. Die Ergebnisse sind aus Fig. 3 zu entnehmen.

### Beispiel 3

### Bestimmung von T₄ nach Reaktionsschema 1c

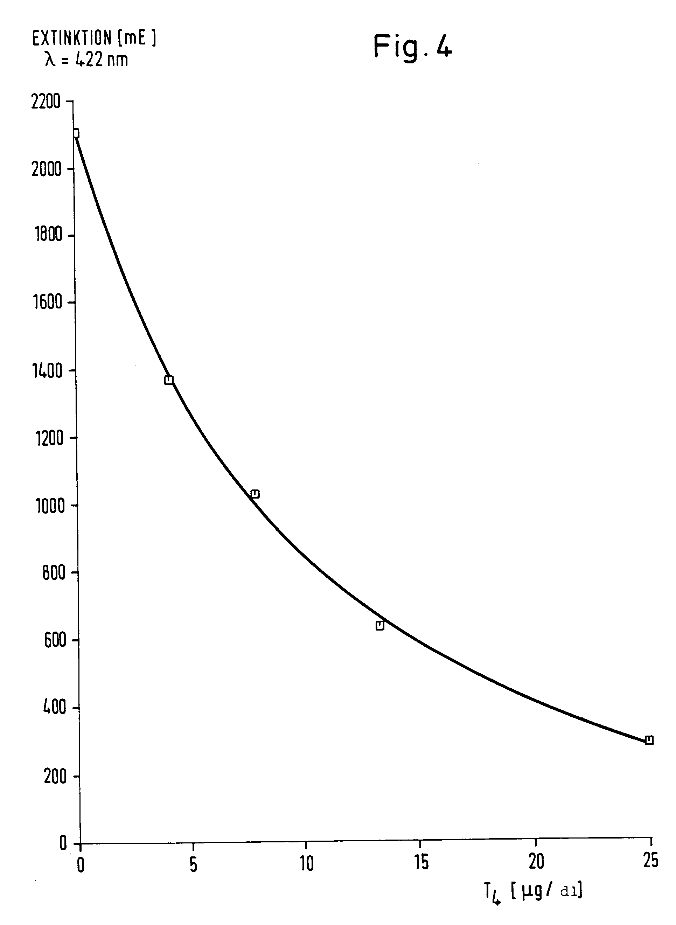

50 µl Probe (mit T₄ aufgestocktes Humanserum) werden mit 480 µl Puffer A und 20 µl einer Lösung eines polyklonalen Antikörpers gegen T₄ (0,1 mg/ml) 30 Minuten bei 25°C in einem streptavidinbeschichteten Polystyrolgefäß inkubiert. Anschließend werden 480 µl Puffer A und 20 µl einer Lösung eines Konjugats aus T₄-Biotin (5 x 10⁻⁷ mol/l, entspricht 10 nmol/l Endkonzentration im Test) und 2,5 U/ml Streptavidin POD-Konjugat (50 mU/Test) zugegeben und 30 Minuten bei 25°C inkubiert. Es wird gewaschen und 1 ml ABTS^{R}-Lösung (9,1 mmol/l) zugegeben, bei 25°C 30 Minuten inkubiert und die optische Dichte bei 422 nm als Maß für den T₄-Gehalt bestimmt. Die Ergebnisse sind aus Fig. 4 zu ersehen.

### Beispiel 4

### Herstellung von T₄-Biotinkonjugat:

N-t-Butyloxycarbonyl-tetrajodthyronin wird über Pentamethylendiamin mit Biotin gekoppelt, wie in Eur. J. Biochem. 131 (1980) 333-338 beschrieben.

## Patentansprüche

1. Verfahren zur Bestimmung einer spezifisch bindefähigen Substanz durch Inkubation der Probelösung mit mindestens drei Rezeptoren R₁, R₂ und R₃, von denen R₁ mit R₂ und R₃ sowie mit der zu bestimmenden Substanz spezifisch bindefähig ist, R₂ die Bindung an die feste Phase vermittelt und R₃ eine Markierung trägt, Abtrennung gebundener von ungebundener Markierung und Messung der Markierung in einer der beiden Phasen, **dadurch gekennzeichnet**, daß als Rezeptor R₁ ein Rezeptor verwendet wird, der mindestens zwei Bindungsstellen, die spezifisch mit einem Epitop der zu bestimmenden Substanz binden, aufweist, als R₂ ein Konjugat aus einem Partner P1 eines spezifisch bindenden Paares und einer Substanz S, die der zu bestimmenden Substanz entspricht oder ein Derivat davon ist und mindestens ein Epitop der zu bestimmenden Substanz aufweist, wobei der Partner P1 entweder an eine feste Phase gebunden ist oder immobilisierbar ist und als R₃ ein Komplex verwendet wird, der mindestens die Substanz S und eine Markierung enthält, verwendet wird.

2. Verfahren nach Anspruch 1, **dadurch gekennzeichnet**, daß als Rezeptor R₁ ein mit der zu bestimmenden Substanz spezifisch bindefähiger Antikörper oder dessen Fab₂-Fragment verwendet wird.

3. Verfahren nach einem der vorhergehenden Ansprüche, **dadurch gekennzeichnet**, daß als Rezeptor R₂ ein Konjugat aus der Substanz S und einem Partner P1 eines spezifisch bindenden Paares verwendet wird und daß als Festphase eine Matrix verwendet wird, an der viele zu P1 komplementäre Partner P2 des spezifisch bindenden Paares gebunden sind.

4. Verfahren nach einem der vorhergehenden Ansprüche, **dadurch gekennzeichnet**, daß als Rezeptor R₂ ein Konjugat aus der Substanz S und einem Partner P1 verwendet wird und daß als Festphase eine Matrix verwendet wird, an der eine Vielzahl Partner P1 gebunden sind, wobei die Immobilisierung nach Inkubation durch Zugabe einer Komponente P2, die mindestens zwei spezifische Bindungsstellen für P1 aufweist, erfolgt.

5. Verfahren nach einem der vorhergehenden Ansprüche, **dadurch gekennzeichnet**, daß als Rezeptor R₃ ein Konjugat aus der Substanz S und einer Markierung verwendet wird.

6. Verfahren nach einem der vorhergehenden Ansprüche, **dadurch gekennzeichnet**, daß als Rezeptor R₃ ein Konjugat aus der Substanz S und dem Partner P1 eines spezifisch bindenden Paares und ein Konjugat aus dem zu P1 komplementären Partner P2 des spezifisch bindenden Paares und einer Markierung verwendet wird.

7. Verfahren nach einem der vorhergehenden Ansprüche, **dadurch gekennzeichnet**, daß als bindendes Paar P1-P2 Biotin-Streptavidin bzw. Avidin, Biotin-Biotinantikörper, Antigen-Antikörper, Hapten-Bindeprotein oder Oligopeptid-Antikörper verwendet werden.

8. Verfahren nach Anspruch 3, **dadurch gekennzeichnet**, daß als Festphase eine Matrix verwendet wird, die aus Kunststoff besteht und an die direkt oder über einen Spacer eine Vielzahl von Bindungspartnern P2 gebunden wird.

9. Reagenz zur Bestimmung einer spezifisch bindefähigen Substanz nach einem der Ansprüche 1 bis 8, **dadurch gekennzeichnet**, daß es Rezeptor R₁, der mindestens zwei für die zu bestimmende Substanz spezifische Bindungsstellen aufweist, Rezeptor R₂, der ein Konjugat aus einem Partner P1 eines spezifische bindenden Paares und einer Substanz S, die der zu bestimmenden Substanz entspricht, oder ein Derivat davon ist und mindestens ein Epitop der zu bestimmenden Substanz aufweist, ist und Rezeptor R₃, der ein Komplex ist, der mindestens eine Markierung und die Substanz S enthält, und physikalisch getrennt davon, eine Festphase, an die P2 immobilisiert ist, enthält.

10. Reagenz zur Bestimmung einer spezifisch bindefähigen Substanz nach einem der Ansprüche 1 bis 8, **dadurch gekennzeichnet**, daß es Rezeptor R₁, der mindestens zwei für die zu bestimmende Substanz spezifische Bindungsstellen aufweist, Rezeptor R₂, der ein Konjugat aus einem Partner P1 eines spezifische bindenden Paares und einer Substanz S, die der zu bestimmenden Substanz entspricht, oder ein Derivat davon ist und mindestens ein Epitop der zu bestimmenden Substanz aufweist, ist und Rezeptor R₃′, der ein Komplex ist, der mindestens eine Markierung und Partner P2 enthält, und physikalisch getrennt davon eine Festphase, an die P2 immobilisiert ist, enthält.

## Claims

1. Process for the determination of a specifically bindable substance by incubation of the sample solution with at least three receptors R₁, R₂ and R₃, of which R₁ is specifically bindable with R₂ and R₃, as well as with the substance to be determined, R₂ brings about the binding to a solid phase and R₃ carries a labelling, separation of bound from unbound labelling and measurement of the labelling in one of the two phases, characterised in that, as receptor R₁, a receptor is used which has at least two binding positions which bind specifically with an epitope of the substance to be determined, as R₂ a conjugate of a partner P₁ of a specifically binding pair and of a substance S which corresponds to the substance to be determined or is a derivative thereof and has at least one epitope of the substance to be determined, whereby the partner P₁ is either bound to a solid phase or is immobilisable and, as R₃, a complex is used which contains at least the substance S and a labelling.

2. Process according to claim 1, characterised in that, as receptor R₁, an antibody specifically bindable with the substance to be determined or its Fab₂ fragment is used.

3. Process according to one of the preceding claims, characterised in that, as receptor R₂, a conjugate of the substance S and a partner P₁ of a specifically binding pair is used and that, as solid phase, a matrix is used to which are bound many partners P₂ of the specifically binding pair complementary to P₁.

4. Process according to one of the preceding claims, characterised in that, as receptor R₂, a conjugate of the substance S and a partner P₁ is used and that, as solid phase, a matrix is used to which a plurality of partners P₁ are bound, whereby the immobilisation takes place after incubation by addition of a component P₂ which has at least two specific binding positions for P₁.

5. Process according to one of the preceding claims, characterised in that, as receptor R₃, a conjugate of the substance S and a labelling is used.

6. Process according to one of the preceding claims, characterised in that, as receptor R₃, a conjugate of the substance S and the partner P₁ of a specifically binding pair and a conjugate of the partner P₂ of the specifically binding pair complementary to P₁ and a labelling is used.

7. Process according to one of the preceding claims, characterised in that, as binding pair P₁-P₂, biotin-streptavidin or avidin, biotin-biotin antibody, antigen-antibody, hapten-binding protein or oligopeptide-antibody are used.

8. Process according to claim 3, characterised in that, as solid phase, a matrix is used which consists of plastics and on which a plurality of binding partners P₂ is bound directly or via a spacer.

9. Reagent for the determination of a specifically bindable substance according to one of claims 1 to 8, characterised in that it contains receptor R₁ which has at least two specific binding positions for the substance to be determined, receptor R₂ which is a conjugate of a partner P₁ of a specifically binding pair and of a substance S which corresponds to the substance to be determined or is a derivative thereof and has at least one epitope of the substance to be determined and receptor R₃ which is a complex which contains a labelling and the substance S and, physically separated therefrom, a solid phase on which P₂ is immobilised.

10. Reagent for the determination of a specifically bindable substance according to one of claims 1 to 8, characterised in that it contains receptor R₁ which has at least two specific binding positions for the substance to be determined, receptor R₂ which is a conjugate of a partner P₁ of a specifically binding pair and of a substance S which corresponds to the substance to be determined or is a derivative thereof and has at least one epitope of the substance to be determined and receptor R₃' which is a complex which contains at least one labelling and partner P₂ and, physically separated therefrom, a solid phase on which P₂ is immobilised.

## Revendications

1. Procédé de détermination d'une substance capable de se lier spécifiquement par incubation de la solution échantillon avec au moins trois récepteurs R₁, R₂ et R₃, parmi lesquels R₁ est capable de se lier spécifiquement à R₂ et R₃ et à la substance à déterminer, R₂ permet la liaison avec la phase solide et R₃ porte un marqueur, séparation du marqueur lié et du marqueur non lié et mesure du marqueur dans l'une des deux phases, caractérisé en ce que l'on utilise comme récepteur R₁ un récepteur qui présente au moins deux sites de liaison qui se lient spécifiquement à un épitope de la substance à déterminer, comme R₂ un conjugué constitué par un partenaire P1 d'une paire qui se lie spécifiquement et par une substance S qui correspond à la substance à déterminer ou qui en est un dérivé et qui présente au moins un épitope de la substance à déterminer, le partenaire P1 étant lié à une phase solide ou immobilisable et l'on utilise comme R₃ un complexe qui contient au moins la substance S et un marqueur.

2. Procédé selon la revendication 1, caractérisé en ce que l'on utilise comme récepteur R₁ un anticorps capable de se lier spécifiquement à la substance à déterminer ou son fragment Fab₂.

3. Procédé selon l'une des revendications précédentes, caractérisé en ce que l'on utilise comme récepteur R₂ un conjugué constitué par la substance S et par un partenaire P1 d'une paire qui se lie spécifiquement et en ce que l'on utilise comme phase solide une matrice à laquelle sont liés de nombreux partenaires P2, complémentaires de P1, de la paire qui se lie spécifiquement.

4. Procédé selon l'une des revendications précédentes, caractérisé en ce que l'on utilise comme récepteur R₂ un conjugué constitué par la substance S et par un partenaire P1 et en ce que l'on utilise comme phase solide une matrice à laquelle sont liés de nombreux partenaires P1, l'immobilisation étant réalisée après incubation par addition d'un composant P2 qui présente au moins deux sites de liaison spécifiques pour P1.

5. Procédé selon l'une des revendications précédentes, caractérisé en ce que l'on utilise comme récepteur R₃ un conjugué constitué par la substance S et par un marqueur.

6. Procédé selon l'une des revendications précédentes, caractérisé en ce que l'on utilise comme récepteur R₃ un conjugué constitué par la substance S et par le partenaire P1 d'une paire qui se lie spécifiquement et un conjugué constitué par le partenaire P2, complémentaire de P1, de la paire qui se lie spécifiquement et par un marqueur.

7. Procédé selon l'une des revendications précédentes, caractérisé en ce que l'on utilise comme paire qui se lie P1-P2 une paire biotine-streptavidine ou avidine, biotine-anticorps antibiotine, antigène-anticorps, haptène-protéine de liaison ou oligopeptide-anticorps.

8. Procédé selon la revendication 3, caractérisé en ce que l'on utilise comme phase solide une matrice qui consiste en matière plastique et à laquelle une multiplicité de partenaires de liaison P2 sont liés directement ou par l'intermédiaire d'un espaceur.

9. Réactif pour la détermination d'une substance capable de se lier spécifiquement selon l'une des revendications 1 à 8, caractérisé en ce qu'il contient un récepteur R₁, qui présente au moins deux sites de liaison spécifiques pour la substance à déterminer, un récepteur R₂, qui est un conjugué constitué par un partenaire P1 d'une paire qui se lie spécifiquement et par une substance S, qui correspond à la substance à déterminer ou qui en est un dérivé et qui présente au moins un épitope de la substance à déterminer, et un récepteur R₃, qui est un complexe qui contient au moins un marqueur et la substance S, et, séparée physiquement des précédents, une phase solide sur laquelle P2 est immobilisé.

10. Réactif pour la détermination d'une substance capable de se lier spécifiquement selon l'une des revendications 1 à 8, caractérisé en ce qu'il contient un récepteur R₁, qui présente au moins deux sites de liaison spécifiques pour la substance à déterminer, un récepteur R₂, qui est un conjugué constitué par un partenaire P1 d'une paire qui se lie spécifiquement et par une substance S, qui correspond à la substance à déterminer ou qui en est un dérivé et qui présente au moins un épitope de la substance à déterminer, et un récepteur R₃', qui est un complexe qui contient au moins un marqueur et le partenaire P2, et, séparée physiquement des précédents, une phase solide sur laquelle P2 est immobilisé.
